# EUROPÄISCHE PATENTSCHRIFT

(11) **EP 1 396 253 B1**
(45) Veröffentlichungstag und Bekanntmachung des Hinweises auf die Patenterteilung: **02.11.2006**
(21) Anmeldenummer: 03018699.3
(22) Anmeldetag: 22.08.2003
(51) Int. Cl.: A61K 6/00, A61K 6/083

(54) **Dentale Polymerfolie**
Polymeric dental sheet
Film dentaire polymerique

(30) Priorität: 06.09.2002 DE 10241434
(43) Veröffentlichungstag der Anmeldung: 10.03.2004
(73) Patentinhaber: Ivoclar Vivadent AG, 9494 Schaan (LI)
(72) Erfinder: Moszner, Norbert, Prof. Dr., 9492 Eschen (LI); Zimmermann, Jörg, 6890 Lustenau (AT); Rheinberger, Volker, Dr., 9490 Vaduz (LI); Besek, Mario, Dr., 8800 Thalwil (CH); Salz, Ulrich, Dr., 88131 Lindau (DE); Fischer, Urs-Karl, 9320 Arbon (CH); Schmidlin, Patrick, Dr., 4616 Kappel SO (CH)
(74) Vertreter: UEXKÜLL & STOLBERG

(56) Entgegenhaltungen:
- EP-A- 0 664 999
- WO-A-95/15740
- WO-A-99/20227
- DE-A- 19 743 897
- US-A- 5 433 941

## Beschreibung

Die vorliegende Erfindung betrifft eine dentale Polymerfolie, die zur Beschichtung von Zahnoberflächen und insbesondere deren Schutz vor Karies eingesetzt werden kann.

Karies ist ein an Prädilektionsstellen der Zahnoberfläche beginnender und in die Tiefe fortschreitender Entkalkungs- und Auflösungsprozess von Schmelz und Dentin, ausgelöst durch bakterielle Säuren bzw. beschleunigt durch diätetische Säuren. Als Prädilektionsstellen gelten Fissuren und Grübchen, Approximalflächen im Zahnkontakt und zervikale Glattflächen. Eine kariöse Läsion beginnt als zunächst nur mikroskopisch sichtbarer Demineralisationsprozess, wobei die Konturoberfläche der betroffenen Zahnhartsubstanzareale vorläufig noch erhalten bleibt. Bei weiterem Fortschreiten des kariösen Prozesses bricht schließlich die noch intakte Oberflächenschicht ein. Eine vollständige Wiederherstellung ist dann nicht mehr möglich, da unter anderem eine Matrixstruktur fehlt, um eine Repräzipitation von Calciumphosphatverbindungen zu ermöglichen.

Die Besiedelung mit pathogenen Keimen (Plaque) ist die Hauptursache für Erkrankungen der Zahnhartsubstanz und des Zahnhalteapparats. Streptokokkus mutans und Lactobazillen sind für den Kariesprozess verantwortlich, Anaerobier wie Prevotella intermedia, Actinobacillus actinomycetemcomitans, Porphyromonas gingivalis u.a. für Gingivitis und Parodontitis.

Die Versiegelung von Fissuren und Grübchen hat sich als effiziente prophylaktische Massnahme erwiesen. Mit einer absolut dichten, adhäsiven Versiegelung auf Polymerbasis werden drei Ziele erreicht: Die Progression bestehender Schmelz- und initialer Dentinläsionen in der Tiefe der Fissur wird gestoppt; der versiegelte Schmelz wird säureunlöslich; die Kariesprädilektionsstelle wird durch Veränderung der Zahnmorphologie eliminiert. Allerdings stellt das Aufbringen der Substanz zur Versiegelung des Zahnes insbesondere im Approximalraum ein Problem dar.

Für die Prävention bzw. Therapie von Karies und Gingivitis werden häufig die z.B. in S.G. Ciancio, "Agents for the management of plaque and gingivitis", J. Dent. Res. 71, 1450 (1992) und P.D. Marsh, "Microbiological aspects of the chemical control of plaque and gingivitis", J. Dent. Res. 71, 1431 (1992) aufgeführten antimikrobiellen Wirkstoffe eingesetzt. Beispiele hierfür sind:

Organische Verbindungen wie z.B. Bisbiguanide, Chlorhexidindigluconat oder -diacetat; quarternäre Ammoniumverbindungen wie Benzalkoniumchlorid und Cetylpyridiniumchlorid; phenolische Verbindungen wie Triclosan, Thymol und Hexetidin und anorganische Ionen wie Silber-, Zink-, Zinn- und Kupferionen.

Angewendet werden diese Wirkstoffe vor allem in Form von Mundwasser, Zahnpasta oder Gel. Nachteilig ist, dass durch die relativ kurze Kontaktzeit für eine klinisch erfolgreiche Therapie eine tägliche, mindestens zweimalige Anwendung erforderlich ist. Dies bedeutet, dass der Behandlungserfolg sehr stark von der Disziplin des Patienten abhängig ist. Zudem sind die eingesetzten Wirkstoffe so unspezifisch, dass auch nicht pathogene Keime abgetötet werden und somit das biologische Gleichgewicht in der Mundhöhle deutlich gestört wird.

Bei der Parodontitistherapie werden in Kombination zur mechanischen Wurzelglättung auch Antibiotika wie z.B. Metronidazol, Amoxicillin oder Doxycyclin systemisch verabreicht. Bei systemischer Gabe ist jedoch mit starken Nebenwirkungen zu rechnen, die vor allem durch die Zerstörung der Darmflora hervorgerufen werden. Aus diesem Grund ist man bei der antibakteriellen Parodontitistherapie dazu übergegangen, die Wirkstoffe lokal in Form von Gelen, oder auch mittels gecoatetem Faden bzw. chipförmigem Wirkstoffträger zu applizieren, wie in G. Greenstein, A. Polson: "The role of local drug delivery in the management of periodontal diseases: a comprehensive review"; J. Periodontol 69, 507 (1998) beschrieben.

Aus der DE 197 43 897 A1 sind Mittel zur Prophylaxe und Therapie von Approximalkaries bekannt, die eine therapeutisch wirksame Menge eines Fluorids enthalten, welches in ein resorbierbares Polymermaterial eingebettet ist. Das Fluorid soll dabei kontinuierlich aus dem Polymermaterial freigesetzt werden. Als weitere Inhaltsstoffe werden Antiplaquemittel oder Antibiotika aufgeführt. Das Mittel wird zwischen den Zähnen (approximal) fixiert, wobei es für einen besseren Halt zusätzlich mit einer Klebeschicht versehen sein kann. Die Prophylaxe und Therapie von Approximalkaries wird also vornehmlich durch die Freisetzung eines Fluorids im Approximalraum erzielt. Allerdings zeigen diese Mittel eine nur unzureichende Haftung an dem Zahn und sie sind aufgrund ihrer Steifheit nur schwierig an die Zahnoberfläche vollständig anformbar.

Aus der US 5,626,866 ist ein Gel als Wirkstoff-freisetzendes System bekannt, wobei dieses Gel auf eine Folie aufgebracht und dann auf die Schleimhaut oder die Haut appliziert wird. Der Wirkstoff wird dann aus dem Gel freigesetzt. Das System ist insbesondere als sogenanntes Nikotinpflaster einsetzbar.

Ein weiterer Lösungsansatz wird in EP 452 446 B1 beschrieben.

Hierbei werden Wirk- und Zusatzstoffe für die Mund- und Zahnhygiene zusammen mit einem Bindemittel zu einer Folie verarbeitet, und diese Folie wird in den Mund eines Patienten eingebracht. Die Bindemittelmischung kann auch auf eine Trägerfolie aufgebracht sein. Die Folie ist nicht für eine längere Verweildauer im Mund vorgesehen und zeigt daher eine nur geringe Haftung an der Zahnoberfläche.

Zusammenfassend kann also festgestellt werden, dass die bekannten Systeme entweder nur schwer auf die zu schützenden Stellen der Zahnoberfläche aufbringbar sind oder wegen Mängeln bei der Anformbarkeit oder wegen ihrer schlechten Hafteigenschaften nicht oder nur unzureichend als vollständiger Schutz der Zahnoberfläche dienen können. Aufgrund des unzureichenden Kontakts sind sie auch häufig nicht in der Lage, den Zahn mit ggf. vorhandenem Wirkstoff in ausreichendem Maße zu versorgen.

Daher liegt der Erfindung die Aufgabe zugrunde, ein Mittel zur Beschichtung von Zahnoberflächen und insbesondere zur Prävention bzw. zur Behandlung von Zahnkaries zur Verfügung zu stellen, bei dem die vorgenannten Nachteile nicht auftreten.

Diese Aufgabe wird überraschenderweise durch die dentale Polymerfolie gemäß den Ansprüchen 1 bis 19 gelöst. Die Erfindung betrifft weiter die Verwendung der Polymerfolie gemäß den Ansprüchen 20 und 21, das Verfahren zur Beschichtung einer Zahnoberfläche gemäß den Ansprüchen 22 und 23 und den Kit gemäß Anspruch 24.

Die erfindungsgemäße dentale Polymerfolie zum Beschichten von Zahnoberflächen zeichnet sich dadurch aus, dass die Polymerfolie polymerisierbare Gruppen aufweist.

Es hat sich überraschenderweise gezeigt, dass das Vorhandensein von noch polymerisierbaren Gruppen in der Polymerfolie zu einer sehr guten Anformbarkeit der Folie an Zahnoberflächen und nach erfolgter Polymerisation dieser Gruppen zu gehärteten Folien mit ausgezeichneten mechanischen Eigenschaften und hervorragender Haftung an den Zahnoberflächen führt. Daher kann die Folie bevorzugt für eine dauerhaften Verbleib im besonders schwer zugänglichen Approximalbereich verwendet werden.

Die Bereitstellung der polymerisierbaren Gruppen in der Folie kann auf verschiedenen Wegen erreicht werden. Beispielsweise kann bei der Herstellung der Folie die Polymerisation bei einem bestimmten Polymerisationsgrad abgebrochen werden, so dass noch reaktionsfähige Monomere in der Folie verbleiben. Auch können verschiedene Monomere mit unterschiedlichen Reaktivitäten verwendet werden, so dass in einem ersten Schritt die reaktiveren Monomere die Folie bilden und in einem zweiten Schritt durch erneute Initiierung die auf den Zahn aufgebrachte Folie ausgehärtet wird. Dies kann auch dadurch erreicht werden, dass verschiedene Monomere benutzt werden, die nach unterschiedlichen Mechanismen polymerisiert werden können, so dass z.B. die Folie durch eine kationische Polymerisation hergestellt wird und anschliessend durch eine radikalische Reaktion ausgehärtet wird. Weiterhin ist es möglich, eine Polymerfolie mit Monomeren zu behandeln, d.h. die Polymerfolie z.B. in Monomeren quellen zu lassen, so dass die Monomere in die Folie aufgenommen werden und so zu einem Gehalt an polymerisierbaren Gruppen führen. Auch können Polymere chemisch modifiziert werden, indem polymerisationsfähige Gruppen eingefügt werden.

Die Einführung von polymerisierbaren Gruppen kann durch polymeranaloge Reaktionen erfolgen. Beispielsweise lassen sich technisch zugängliche synthetische Polymere verwenden, die dann chemisch modifiziert werden. Beispiele hierfür sind: Polyvinylalkohol, Vinylalkohol-Copolymere z.B. von Ethen oder Vinylacetat, sowie Polyvinylacetale, in die durch Umsetzung der vorhandenen OH-Gruppen mit 2-Isocyanatoethylmethacrylat oder Methacrylsäurechlorid bzw. -anhydrid polymerisationsfähige Methacrylat-Gruppen einführbar sind. Gleichfalls lassen sich auch beliebige Copolymere der Acryl- oder Methacrylsäure z.B. mit Acryl- oder Methacrylsäureestern, Styrol, Vinylacetat, Acrylnitril oder Acryl- bzw. Methacrylamid mit Glycidylmethacrylat bzw. 2-Isocyanatoethylmethacrylat umsetzen, wobei die polymerisationsfähigen Gruppen durch Ester- bzw. Amidgruppen seitenständig an die Primärkette gebunden sind. Analog ist auch eine chemische Modifizierung von Biopolymeren möglich. Beispielsweise lassen sich Cellulose oder Cellulose-Derivate, wie Ester oder Ether, die für ihre guten filmbildenden Eigenschaften bekannt sind, durch Umsetzung mit 2-Isocyanatoethylmethacrylat oder Methacrylsäurechlorid bzw. -anhydrid derivatisieren.

Es ist bevorzugt, dass die polymerisierbaren Gruppen zumindest teilweise und insbesondere überwiegend Acrylat- oder Methacrylat-Gruppen sind.

Die polymerisierbaren Folien können auch durch Polyaddition von Monomeren bzw. Oligomeren oder Prepolymeren, die polymerisationsfähige Gruppen tragen, so hergestellt werden, dass zumindest ein Teil dieser Gruppen für die weitere Aushärtung der Polymerfolie verfügbar ist.

Die erfindungsgemäße dentale Polymerfolie ist vorzugsweise auf der Basis von Polyurethanen, Michael-Additionsharzen oder anderen Polyaddukten aufgebaut.

Als Polyurethane werden insbesondere die Produkte der Reaktion von Diisocyanaten, wie z.B. Toluylendiisocyanat, Methylendiphenyldiisoyanat, 2,2,4-Trimethylhexamethylendiisocyanat oder Isophorondiisocyanat oder den daraus hergestellten oligomeren Polyisocyanaten, mit geeigneten Polyolen eingesetzt, wie Ethylenglycol, Propylenglycol, Glycerin, Trimethylolpropan, Pentaerythrit, Sorbit, Polyethylenglycol, Polypropylenglycol oder entsprechende Copolymeren oder daraus hergestellten di- oder trifunktionellen Polyolen.

Als Katalysatoren für diese Reaktion kommen vornehmlich zinnorganische Verbindungen oder tertiäre Amine in Frage.

Zur Einführung der polymerisierbaren Gruppen werden die Isocyanate zusätzlich mit polymerisierbaren Hydroxy-Verbindungen, insbesondere 2-Hydroxyethyl(meth)acrylat, Hydroxypropylmethacrylat, N-(2-Hydroxyethyl)(meth)acrylamid, Glycerinmono- und -dimethacrylat oder Bis-GMA, umgesetzt.

Durch die gezielte Auswahl der Isocyanat- und Alkoholkomponenten lassen sich die Flexibilität, die Festigkeit, der Vernetzungsgrad, die Quelleigenschaften gegenüber verschiedenen Lösungsmitteln und die Hydrophilie der Folien sowohl vor als auch nach der abschliessenden Polymerisation variieren.

Bevorzugt in der erfindungsgemäßen Polymerfolie eingesetzte Michael-Reaktionsharze sind die Umsetzungsprodukte von multifunktionellen Acrylaten mit di- oder multifunktionellen Acetoacetaten. Beispiele für geeignete Acrylate sind Ethylenglycoldiacrylat, Hexandioldiacrylat, Tripropylenglycoldiacrylat, ethoxyliertes Bisphenol-A-Diacrylat,Polyethylenglycol-200-diacrylat, Trimethylolpropantriacrylat, Pentaerythrittetraacrylat. Diese Acrylate lassen sich insbesondere mit tri- oder tetrafunktionellen Acetoacetaten, wie z.B. Trimethylolpropan- und Glycerintrisacetoacetat sowie Pentaerythrit-tetrakis-acetoacetat zu Netzwerkpolymeren umsetzen. Als Katalysatoren für die Bildung der Michael-Additionsharze werden vorzugsweise Alkalimetallhydroxide, z.B. KOH, Tetraalkylammoniumhydroxide, z.B. Tetrabutylammoniumhydroxid, insbesondere aber bicyclische Amidine, wie 1,5-Diazabicyclo [4.3.0] -5-nonen oder 1, 8-Diazabicylo[5.4.0]-7-undecen, und Guanidine, vor allem Tetramethylguanidin, eingesetzt. Polyadditionsprodukte mit nicht umgesetzten polymerisierbare Gruppen werden erhalten, wenn die multifunktionellen Acrylate im Überschuss eingesetzt werden, oder Acetoacetate mit polymerisierbaren Gruppen, wie z.B. 2-Acetoacetoxyethylmethacrylat, verwendet werden, oder Lösungen der multifunktionellen Acrylate in Mono- oder Dimethacrylaten bzw. deren Mischungen eingesetzt werden. Durch die Struktur und Funktionalität der Acetoacetate und Acrylate lassen sich Elastizität, Festigkeit und die Filmbildungseigenschaften der erhaltenen Polyaddukte gezielt variieren.

Bevorzugt in der erfindungsgemäßen Polymerfolie eingesetzte andere Polyaddukte sind Epoxid-Amin-Addukte, Epoxid-Anhydrid-Addukte sowie Polysiloxan-Addukte. Weitere geeignete andere Polyaddukte sind über die Thiol-En-Reaktion zugänglich.

Die Reaktivität und die Eigenschaften der erfindungsgemäßen Polymerfolie lassen sich durch Verwendung der folgenden Additive bei der Herstellung der Folie gezielt beeinflussen.

Radikalisch polymerisierbare Verbindungen können als Additive eingesetzt werden, um die Festigkeit und den E-Modul der Folie zu erhöhen oder die Wasseraufnahme zu verringern. Hierfür eignen sich insbesondere (Meth)acrylate, Styrol und Styrolderivate, Allylverbindungen oder Vinylcyclopropane, wobei (Meth)acrylate besonders bevorzugt sind. Bevorzugt werden monofunktionelle Monomere, wie z.B. Methyl-, Ethyl-, Butyl-, Benzyl-, Furfuryl- oder Phenyl(meth)acrylat, und die als Vernetzermonomere bekannten mehrfunktionellen Acrylate bzw. Methacrylate, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenclycoldi(meth)acrylat, Trimethylolpropantri(meth)acrylat, Pentaerythrit-tetra(meth)acrylat, sowie Butandioldi(meth)acrylat, Glycerindimethacrylat, 1,10-Decandioldi(meth)acrylat oder 1,12-Dodecandioldi(meth)acrylat, verwendet.

Oligomere und Polymere, die endständige und/oder seitenständige radikalisch polymerisierbare Gruppen tragen, können als Additive eingesetzt werden, um die Flexibilität der Folie und deren Substrathaftung zu verbessern. Hierfür eignen sich insbesondere α,ω-(meth)acryloyl-terminierte Polyester-, Polyether-, Polyepoxid-Amin- oder Polyurethan-Telechele oder Kieselsäurepolykondensate, wie sie durch hydrolytische Kondensation von Silanen erhältlich sind. Solche Kieselsäurepolykondensate sind beispielsweise in der DE 44 16 857 C1 oder DE 41 33 494 C2 beschrieben. Als radikalisch polymerisierbare Gruppen sind Methacryl- oder Acrylgruppen bevorzugt.

Kationisch polymerisierbare Verdünner- oder Vernetzermonomere können als Additive eingesetzt werden, um die Aushärtung der Folie zu beschleunigen. Hierfür eignen sich insbesondere Glycidylether oder cycloaliphatische Epoxide, cyclische Ketenacetale, Vinylether, Spiro-orthocarbonate, Oxetane oder bicyclische Orthoester. Bevorzugte Beispiele sind Triethylenglycoldivinylether, Cyclohexandimethanol-divinylether, 2-Methylen-1,4,6-trioxaspiro[2.2]nonan,3,9-Dimethylen-1,5,7,11-tetraoxaspiro[5.5]undecan, 2-Methylen-1,3-diox-epan, 2-Phenyl-4-methylen-1,3-dioxolan, Bisphenol-A-diglycidylether, 3,4-Epoxy-cyclohexylmethyl-3,4-epoxycyclohexancarboxylat, Bis-(3,4-epoxycyclohexylmethyl)adipat, Vinylcyclohexendioxid, 3-Ethyl-3-hydroxymethyloxetan, 1,10-Decandiylbis(oxymethylen)bis(3-ethyl-oxetan) oder 3,3-(4-Xylylendioxy)-bis-(methyl-3-ethyloxetan).

Kationisch polymerisierbare Kieselsäurepolykondensate können als Additive eingesetzt werden, um die Abrasionsfestigkeit und Härte der Folie zu verbessern. Hierfür eignen sich Kieselsäurepolykondensate, die kationisch polymerisierbare Gruppen, bevorzugt z.B. Epoxid-, Oxetan-, Spiroorthoester oder Vinylethergruppen tragen. Die Synthese dieser Kieselsäurepolykondensate kann beispielsweise durch hydrolytische Kondensation von Silanen erfolgen, wie sie z.B. in der DE 41 33 494 C2 oder der US 6,096,903 beschrieben sind.

Es sind erfindungsgemäße Polymerfolien bevorzugt, bei denen zumindest ein Teil der polymerisierbaren Gruppen radikalisch und/oder kationisch polymerisierbar ist.

Zur Initiierung der Polymerisation der polymerisierbaren Gruppen enthält die Polymerfolie vorzugsweise Initiatoren für eine thermische, photochemische oder redoxinduzierte Polymerisation.

Diese Initiatoren können insbesondere in mikroverkapselter Form vorliegen. Dadurch wird es ermöglicht, die Aushärtungsreaktion durch Druck oder Ultraschall zu starten.

Zur Initiierung der radikalischen Polymerisation werden vorzugsweise thermische und/oder Photoinitiatoren eingesetzt. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat, auch in Kombination mit Barbitur- oder Sulfinsäurederivaten. Weitere thermische Initiatoren sind Azo-bis-isobutyroethylester, Azo-bis-isobutyronitril, Benzpinakol oder 2,2-Dimethylbenzpinakol. Beispiele für geeignete Photoinitiatoren sind Benzophenon, Benzoin sowie deren Derivate oder α-Diketone oder deren Derivate wie 9,10-Phenanthrenchinon, Diacetyl oder 4,4-Dichlorbenzil. Bevorzugt werden Campherchinon und 2,2-Methoxy-2-phenyl-acetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N- Dimethyl-sym.-xylidin oder Triethanolamin, eingesetzt. Acylphosphine, wie z.B. 2,4,6-Trimethylbenzoyldiphenyl- oder Bis(2,6-dichlor-benzoyl)-4-N-propylphenylphosphinoxid sind besonders bevorzugt.

Für die Aushärtung von kationisch polymerisierbaren Gruppen sind Diaryliodonium- oder Triarylsulfoniumsalze, wie z.B. Triphenylsulfoniumhexafluorophosphat oder -hexafluoroantimonat besonders bevorzugt. Dabei ist vorteilhaft, dass diese Initiatoren sich auch zur Aushärtung der radikalisch polymerisierbaren Gruppen der Folien eignen.

Zur Verstärkung kann die erfindungsgemäße Polymerfolie organische oder anorganische Füllstoffe enthalten. Zu diesem Zweck werden diese Füllstoffe in der Regel bei der Herstellung der Polymerfolie eingearbeitet. Die Einarbeitung von faserförmigen Füllstoffen kann dabei je nach Anwendung isotrop oder anisotrop erfolgen. Bevorzugte anorganische teilchenförmige Füllstoffe sind amorphe kugelförmige Materialien auf der Basis von Oxiden, wie ZrO₂ und TiO₂, oder auf Basis von Mischoxiden aus SiO₂, ZrO₂ und/oder TiO₂, und besonders bevorzugt mikrofeine oder nanopartikuläre Füllstoffe, wie Silsesquioxane oder Metalloxidcluster, pyrogene Kieselsäure oder Fällungskieselsäure mit einer durchschnittlichen Teilchengrösse von 1 bis 500 nm sowie röntgenopake Füllstoffe, wie z.B. Ytterbiumtrifluorid, Bariumsulfat, Lanthan- oder Tantaloxid. Darüber hinaus können auch sehr kurze Glasfasern, Whisker oder Schichtsilikate, z.B. Glimmerpartikel, als Füllstoffe verwendet werden. Besonders vorteilhaft ist es, wenn die Füllstoffe mit geeigneten Haftvermittlern oberflächenmodifiziert worden sind.

Zur Verhinderung einer vorzeitigen Polymerisation der vorhandenen polymerisierbaren Gruppen enthält die erfindungsgemäße Polymerfolie bevorzugt geeignete Polymerisationsinhibitoren. Dafür eignen sich aerobe Inhibitoren, wie aromatische Phenole, z.B. Hydrochinonmonomethylether (MEHQ) oder 2,5-di-tert.-butyl-4-methyl-phenol (BHT), und insbesondere anaerobe Inhibitoren, wie sekundäre aromatische Amine z.B. Phenothiazin, N,N'-Diphenylphenylendiamin oder stabile Radikale, z.B. das 2,2,6,6-Tetramethylpiperidin-1-oxyl-Radikal (TEMPO).

Zur Stabilisierung der ausgehärteten Polymerfolie kann diese vorzugsweise ein oder mehrere Antioxidantien, z.B. Alkylphenole, Hydroxyphenylpropionate, Hydroxybenzyl-Verbindungen oder Alkylidenbisphenole, und/oder Lichtschutzmittel, wie UV-Absorber vom Hydroxybenzophenon- oder 2-(2-Hydroxyphenyl)-benzotriazol-Typ, enthalten.

Um der dentalen Polymerfolie ein dem Zahn entsprechendes Aussehen zu verleihen, kann sie ausserdem einen oder mehrere Farbstoffe und/oder Pigmente enthalten. Hierbei kommen vor allem Pigmente, wie TiO₂ und ZnO, oder Farbpigmente, wie chromophore Xerogele, oder Farbstoffe, wie beispielsweise Azo-, Phenothiazin- oder Anthrachinonfarbstoffe zur Anwendung. Aber auch die Verwendung von thermochromen Farbstoffen, wie Chromazone Blue (Eckart), RT31 (Kelly Corp.) oder pH-sensitiven Farbstoffen, wie beispielsweise Thymolblau, Kongorot, Alizarin oder Phenolphthalein, ist möglich.

Zur Verbesserung der Haftung auf der Zahnoberfläche kann die erfindungsgemäße Polymerfolie vorzugsweise einen Haftvermittler aufweisen. Dieser kann in der Polymerfolie enthalten oder auf einer Seite aufgeschichtet sein. Bevorzugt ist die dentale Polymerfolie auf der der Zahnoberfläche zugewandten Seite mit einem Haftvermittler für die Zahnhartsubstanz beschichtet. Dabei sind vor allem polymerisationsfähige Carbonsäuren, Phophonsäuren oder Dihydrogenphosphorsäuremonoester als Haftvermittler einsetzbar. Bevorzugte Beispiele sind: Acrylsäure, Methacrylsäure, 4-Methacryloyloxyethyloxycarbonyl-phthalsäure (4-MET) oder dessen Anhydrid (4-META), 10-Methacryloyloxydecamethylen-malonsäure, 4-Methacryloylaminosalicylsäure, der Phosphorsäureester von Pentaacryloyldipentaerythritol, 2-Methacryloyloxydecamethylenphosphorsäuremonoester (MDP), 4-Vinylbenzylphosphonsäure oder 2-[4-(Dihydroxyphosphoryl)-2-oxabutyl]acrylsäureethylester. Mit solchen Säuremonomeren lassen sich auch Polymerfolien herstellen, die zu einem Ionenaustausch befähigt sind.

Die dentale Polymerfolie kann ausserdem ein Antihaftadditiv aufweisen. Sie ist bevorzugt auf der der Zahnoberfläche abgewandten Seite mit einem Antihaftadditiv für Plaque beschichtet, um auf diese Weise die unerwünschte Ansammlung von Plaque oder eine Verfärbung zu verhindern. Geeignete Antihaftadditive sind fluorierte Monomere, wie z.B. perfluorierte Alkylmethacrylate.

Darüberhinaus kann die dentale Polymerfolie noch eine oder mehrere Wirkstoffe enthalten. Als Wirkstoffe kommen vornehmlich antimikrobielle Verbindungen oder Antibiotika in Frage.

Besonders bevorzugt sind solche Wirkstoffe, die über eine entsprechende funktionelle Gruppe im Molekül mit den zum Aufbau der Polymerfolie verwendeten Monomeren copolymerisieren können.

Somit werden sie polymergebunden immobilisiert, wodurch eine langfristige Ausrüstung der Polymerfolie und insbesondere ihrer Oberfläche erzielt wird. Dies führt dazu, dass anhaftende Keime abgetötet werden oder zumindest deutlich an Aktivität verlieren. Entsprechende polymerisierbare Wirkstoffe sind z.B. in EP-A-0 537 774, EP-A-0 705 590 oder DE-A-196 54 897 beschrieben.

Zu den verwendbaren antimikrobiellen Wirkstoffen zählen vor allem die organischen Verbindungen Thymol, Triclosan, Cetylpyridiniumchlorid, Chlorhexidindiacetat sowie anorganische Verbindungen wie Zinkchlorid und Zinnfluorid. Als Antibiotika können insbesondere Metronidazol, Minocyclin, Doxycyclin und Tetracyclin zur Anwendung kommen. Die Wirkstoffe sind bevorzugt in einer Konzentration von 0,01 bis 10 Gew.-% in der erfindungsgemäßen Polymerfolie enthalten.

Weiterhin kann die erfindungsgemäße Polymerfolie auch noch andere Additive enthalten, wie z.B. Aromastoffe, Weichmacher, optische Aufheller oder Effektstoffe.

Die erfindungsgemäße Polymerfolie kann aufgrund ihrer Elastizität und plastischen Verformbarkeit einfach durch den Anwender direkt auf den ausgewählten Bereich eines Zahnes aufgebracht werden. Zur vereinfachten Handhabung ist die Polymerfolie vorzugsweise ablösbar auf einer Trägerfolie gehalten, die insbesondere lichtdurchläsig ist, um eine anschließende Photopolymerisation nicht zu behindern.

In einer bevorzugten Ausführungsform kann die erfindungsgemäße Polymerfolie auch aus mehreren Schichten aufgebaut sein, wobei die einzelnen Schichten spezielle Eigenschaften aufweisen. Einerseits kann die innere, zum Zahn weisende Schicht hydrophil sein und auch Monomere bzw. Funktionalitäten aufweisen und sich damit durch eine besondere Affinität zur Zahnoberfläche auszeichnen, so dass sogar auf den Einsatz eines zusätzlichen Adhäsivs zur Befestigung an der Zahnoberfläche verzichtet werden kann. Andererseits kann die äußere Schicht dagegen hydrophob sein und eine geringe Plaqueaffinität besitzen.

Durch die Flexibilität der noch nicht ausgehärteten Polymerfolie lässt sich diese problemlos an die gekrümmte Zahnoberfläche im Approximalraum anpassen. Zur besseren Zugänglichkeit des Approximalraums wird häufig ein sogenannter Zahnspreizer verwendet, mit dessen Hilfe der Abstand zwischen den betreffenden Zähnen vergrössert und damit das Einbringen der Polymerfolie unterstützt werden kann. Die Polymerfolie kann vom Anwender selbst an die erforderliche Grösse angepasst werden oder es erfolgt die Verwendung von vorgefertigten Folienstücken, die vorzugsweise eine ovale Fläche aufweisen. Danach wird regelmäßig so vorgegangen, dass das Folienstück direkt auf die gereinigte und/oder konditionierte Zahnoberfläche aufgebracht, mit einem geeigneten zahnärztlichen Instrument provisorisch gehalten bzw. fixiert und und schließlich insbesondere mittels Lichtpolymerisation ausgehärtet wird.

Zur Unterstützung der Anpassung kann die Polymerfolie mit Hilfe der vorzugsweise vorgesehenen Trägerfolie auf die Zahnoberfläche aufgebracht werden, wobei nach Polymerisation der polymersierbaren Gruppen und damit der Härtung der Polymerfolie die Trägerfolie wieder entfernt wird.

Besonders einfach läßt sich die Polymerfolie mit Hilfe einer Trägerfolie an die Zahnoberfläche im Approximalraum anpassen, die als aufblasbarer Folienbeutel gestaltet ist. Beim Aufblasen presst das entstehende "Kissen" der Trägerfolie die Polymerfolie im Zwischenraum optimal auf die zu versiegelnde Fläche. Bei Einsatz von einer lichtdurchlässigen Trägerfolie kann diese während einer Lichtpolymerisation im Approximalraum verbleiben. Sie wird erst nach der gewünschten Aushärtung entfernt.

Weiter ist es möglich die Polymerfolie mittels eines Adhäsivs auf der ausgewählten Zahnoberfläche zu befestigen. Eine solche adhäsive Aufbringung kann entweder mittels der sogenannten "Säure-Ätz-Technik" oder mittels eines "self-etching Primer" erfolgen.

Bei der "Säure-Ätz-Technik" wird in einem ersten Schritt mit einer ca. 35%igen Phosphorsäurelösung oder einem entsprechenden Gel die Dentin- und/oder Zahnschmelzoberfläche konditioniert und anschließend in einem zweiten Schritt ein geeignetes Adhäsiv, wie z.B. das Schmelz/Dentin-Adhäsiv Excite® der Fa. Ivoclar Vivadent AG,) auf die Oberfläche aufgebracht. Diese Vorbehandlung führt dazu, dass schließlich ein kovalenter Verbund zwischen der Zahnoberfläche einerseits und der aufgebrachten Polymerfolie andererseits erzielt wird, indem eine Copolymerisation von insbesondere (Meth)acrylatgruppen statfindet, die sowohl im Adhäsiv als auch in der Polymerfolie vorhanden sind.

Hierin zeigt sich auch der besondere Vorteil der erfindungsgemäßen Polymerfolie, die durch ihre Flexibilität nicht nur eine optimale Anpassung an die Zahnoberfläche gestattet, sondern durch die in ihr vorhandenen reaktiven, d.h. polymerisierbaren Gruppen auch kovalente Bindungen zur Zahnoberfläche herstellen kann.

Bei der Verwendung einer Reaktionshaftgrundierung, d.h. eines "self-etching Primers", übernimmt diese die Funktion der Phosphorsäure zur Konditionierung der Zahnoberfläche. Üblicherweise sind solche Reaktionshaftgrundierungen derart aufgebaut, dass sie Säuremonomere enthalten, wie z.B. die in der DE 27 11 234 beschriebenen polymerisierbaren Phosphorsäure- oder Phosphonsäuremethacrylate. Diese wirken auf die Zahnoberfläche vergleichbar zur Phosphorsäure, werden jedoch nach der erforderlichen Einwirkzeit nicht abgespült, sondern werden in einem zweiten Schritt in das Adhäsiv einpolymerisiert.

Die Erfindung betrifft somit auch die Verwendung der erfindungsgemäßen dentalen Polymerfolie zur Beschichtung von Zahnoberflächen und insbesondere zu deren Versiegelung und damit zur Behandlung von oder Schutz vor Karies. Besonders bevorzugt erfolgt dabei eine Behandlung von Zahnoberfläche im Approximalraum. Die Verwendung erfolgt dabei insbesondere dadurch, dass die Polymerfolie auf die Zahnoberfläche aufgebracht und durch Verformen an diese angepasst wird, und die polymerisierbaren Gruppen der aufgebrachten und angepassten Polymerfolie polymerisiert werden.

Die Erfindung stellt weiterhin ein Verfahren zur Beschichtung einer Zahnoberfläche zur Verfügung, bei dem die Polymerfolie auf die Zahnoberfläche aufgebracht und durch Verformen an diese angepasst wird, und die polymerisierbaren Gruppen der aufgebrachten und angepassten Polymerfolie polymerisiert werden.

Dabei ist ein Verfahren bevorzugt, bei dem zur Beschichtung einer Zahnoberfläche im Approximalraum der Approximalraum mittels eines Spreizers gespreizt wird, die zu beschichtende Oberfläche gereinigt und mittels Ätztechnik oberflächenkonditioniert wird, wonach ein Adhäsiv auf die so vorbereitete Zahnfläche aufgebracht und die erfindungsgemäße Polymerfolie in den Approximalraum eingebracht und an die zu versiegelnde Zahnoberfläche angelegt und deren polymerisierbare Gruppen nach Anformung polymerisiert werden, um eine Versiegelung zu ergeben.

Ausserdem betrifft die Erfindung einen Kit, der die erfindungsgemäße Polymerfolie zur Versiegelung und zum mechanischen Schutz der Zahnoberfläche und ein Adhäsiv zur Fixierung der Polymerfolie auf der Zahnoberfläche aufweist.

Die Erfindung wird im folgenden anhand von Beispielen näher erläutert.

### Beispiele

In den folgenden Beispielen 1 bis 4 erfolgte die Herstellung der Polymerfolie durch vernetzende Micheal-Addition von Pentaerythritoltetraacetoacetat (PETAA) mit entweder 1,6-Hexan-dioldiacrylat (HDDA) oder Pentaerythrittetraacrylat (PETA), wobei jeweils 1,5-Diazabicyclo[4.3.0]-5-nonen (DBN) als Katalysator verwendet wurde. Die folgenden weitere Komponenten wurden ggf. zugegeben.
- Photoinitiatorsystem, bestehend aus Campherchinon (CC) und 4-Dimethylaminobenzoesäureethylester (DABE),
- photopolymerisierbare Dimethacrylate, wie Triethylenglycoldimethacrylat (TEGDMA) oder 1,10-Decandiolmethacrylat (D3MA),
- Füllstoffe wie Aerosil OX-50 silanisiert oder Aerosil DT-4 (pyrogenes Siliciumdioxid, das mit 3-Methacryloyloxypropyltrimethoxysilan silanisiert wurde) und/oder Ytterbiumtrifluorid (YbF₃).

Aus den Komponenten wurden jeweils zwei miteinander zu mischende Zusammensetzungen hergestellt, die als Basis-Mischung und Katalysator-Mischung bezeichnet wurden.

### Beispiel 1

Basis- und Katalysator-Mischung wurden aus den nachstehend angegebenen Komponenten unter Rühren hergestellt (Mengen in Gew.-%):

| | Basis-Mischung | Katalysator-Mischung |
|---|---|---|
| PETAA | - | 49.7 |
| HDDA | 47.7 | - |
| DBN | - | 1.1 |
| CC | 0.6 | - |
| DABE | 1.0 | - |
| TEGDMA | 50.7 | 49.2 |
| | 100.0 | 100.0 |

Zur Herstellung der Polymerfolien wurden Basis- und Katalysator-Mischung jeweils im Gewichtsverhältnis 1:1 gemischt, zwischen zwei Polyesterfolien mit 0.1 mm Stahlbändern als Distanzhalter verpresst und 24 h lang bei 50 °C gehalten, um die gewünschte Umsetzung zu erzielen.

Nach dem Entfernen der Deckfolien und Distanzhalter konnten die die erzeugten Polymerfolien auf die gewünschten Abmessungen zugeschnitten werden. Die so hergestellten Polymerfolien waren farblos und wiesen keine Schmierschicht auf. Eine anschliessende Aushärtung mit einer Dentallichtquelle (Spectramat®, Ivoclar Vivadent AG) ergab nach 2.* 3 Minuten Bestrahlungszeit farblose, stabile und biegbare Folien.

Die Messung der Härte der Polmyerfolie nach Michael-Reaktion und nach anschließender Härtung führte zu folgendem Ergebnis.

| | |
|---|---|
| Folie nach Michael-Reaktion (24 h, 50 °C) | - |
| Folie nachgehärtet (2 * 3 min. Spektramat® ) | 78 Shore D |

### Beispiel 2

Basis- und Katalysator-Mischung wurden aus den nachstehend angegebenen flüssigen Komponenten unter Rühren hergestellt (Mengen in Gew.-%):

Aerosil OX-50 silanisiert und YbF₃ wurden in die Basis- oder Katalysator-Mischung per Hand eingerührt und danach über einen 3-Walzenstuhl (Fa. Exakt) homogenisiert.

| | Basis-Mischung | Katalysator-Mischung |
|---|---|---|
| PETAA | - | 22.7 |
| PETA | 16.9 | - |
| DBN | - | 0.5 |
| CC | 0.1 | - |
| DABE | 0.2 | - |
| D3MAt | 22.8 | 16.8 |
| Aerosil OX-50 silanisiert | 40.0 | 40.0 |
| YbF₃ | 20.0 | 20.0 |
| | 100.0 | 100.0 |

Die Herstellung der Polymerfolien erfolgte analog Beispiel 1.

Die hergestellten Polymerfolien mit polymerisierbaren Gruppen waren opak und wiesen keine Schmierschicht auf. Die anschließende photochemische Aushärtung führte zu stabilen und biegbaren Folien. Nach einer Lagerung für 72 Stunden bei 37 °C in entionisiertem Wasser konnten keine Veränderungen der Folien-Eigenschaften festgestellt werden. Die Wasseraufnahme betrug lediglich 1,85 Gew.-%.

### Messergebnis der Härtemessung (Mittelwerte aus 5 Messungen)

| | |
|---|---|
| Folie nach Michael-Reaktion (24 h, 50 °C) | - |
| Folie nachgehärtet (2 * 3 min. Spektramat®) | 92 Shore D |
| Lagerung in Wasser (72 h, 37 °C) und nachfolgende Trocknung (24 h, 37 °C) | 91 Shore D |
| Wasseraufnahme (in %) | + 1,85 |

### Beispiel 3

Basis- und Katalysator-Mischung wurden aus den nachstehend angegebenen Komponenten hergestellt:

| | Basis-Mischung | Katalysator-Mischung |
|---|---|---|
| PETAA | - | 46.6 |
| PETA | 52.3 | - |
| DBN | - | 1.0 |
| CC | 0.6 | - |
| DABE | 1.0 | - |
| TEGDMA | 46.1 | 52.4 |
| | 100.0 | 100.0 |

Die Herstellung und Aushärtung der Folien erfolgte analog Beispiel 1. Es wurden dabei farblose, stabile und biegbare Folien erhalten, die auf den Oberflächen keinerlei Schmierschichten aufwiesen.

### Messergebnis der Härtemessung

| | |
|---|---|
| Folie nach Michael-Reaktion (24 h, 50 °C) | 56 Shore D |
| Folie nachgehärtet (2 * 3 min. Spektramat® ) | 84 Shore D |

### Beispiel 4

Basis- und Katalysator-Mischung wurden aus den nachstehend aufgeführten Komponenten (Mengen in Gew.-%) analog Beispiel 2 hergestellt.

| | Basis-Mischung | Katalysator-Mischung |
|---|---|---|
| PETAA | 32.6 | - |
| PETA | 36.5 | - |
| DBN | - | 0.8 |
| CC | - | 0.4 |
| DABE | - | 0.7 |
| D3MA | - | 69.0 |
| Aerosil DT-4 | 30.9 | 29.1 |
| | 100.0 | 100.0 |

Die Herstellung und Aushärtung der Folien erfolgte analog Beispiel 2. Es ergaben sich opake stabile und biegbare Folien, deren Eigenschaften sich nach Wasserlagerung nicht veränderten.

### Messergebnis der Härtemessung (Mittelwerte aus 5 Messungen)

| | |
|---|---|
| Folie nach Michael-Reaktion 24 h, 50 °C) | - |
| Folie nachgehärtet (2 * 3 min. Spektramat®) | 90 Shore D |
| Lagerung in Wasser (72 h, 37 °C) und nachfolgende Trocknung (24 h, 37 °C) | 88 Shore D |
| Wasseraufnahme (in %) | + 2.05 |

### Beispiel 5

Dieses Beispiel zeigt Polymerfolien auf Basis von Polyurethanen.

Die Herstellung der Polymerfolien erfolgte durch Additionsreaktion eines Triisocyanates an Di- und Monohydroxy-Verbindungen, die weitere polymerisierbare Acryl- oder Methacrylgruppen enthalten. Als Katalysator für die Bildung des Polyurethans wurde Metatin 802 (Di-n-octylzinndiacetat) eingesetzt, wobei die anschliessende Härtung unter Verwendung eines Photoinitiatorsystem aus Campherchinon (CC), EMBO ((p-Dimethylamino)-benzolsäureethylester) und Lucirin TPO (2,4,6-Trimethylbenzoyldiphenylphosphinoxid) und durch Bestrahlung mit einem Spectramat® erfolgte. Als Füllstoff wurde Aerosil DT-4 verwendet:

| | |
|---|---|
| GDMA | 22.0 |
| Bis-GMA | 22.0 |
| LS 2294, Asymmetrisches Diisocyanat-Trimerisat mit Iminooxadiazindion-Struktur, erhältlich ausgehend von Hexamethyldiisocyanat | 35.1 |
| CC | 0.1 |
| EMBO | 0.4 |
| Lucirin TPO | 0.5 |
| Aerosil DT-4 | 19.8 |
| Metatin 801 | 0.1 |
| | 100.0 |

Zur Folienherstellung wurden jeweils 10 g der oben genannten Zusammensetzung mit Metatin versetzt und zwischen zwei Parafilm-Folien mit 0,1 mm Stahlbändern als Distanzhalter verpresst. Dann ließ man die Mischung 15 Stunden bei Raumtemperatur reagieren. Die so hergestellte Polymerfolie war opak und hatte keine Schmierschicht auf der Oberfläche. Eine anschliessende Polymerisation für 2 * 2 min. Bestrahlung mittels des Spectramats® (Fa. Ivoclar Vivadent AG) ergab stabile und biegbare Folien, die im Wasser sehr beständig waren.

### Messergebnis der Härtemessung (Mittelwerte aus 6 Messungen)

| | |
|---|---|
| Folie nach Reaktion 24 h, 50 °C) | 26 Shore D |
| Folie nachgehärtet (2 * 3 min. Spektramat®) | 82 Shore D |
| Lagerung in Wasser (72 h, 37 °C) und nachfolgende Trocknung (24 h, 37 °C) | 80 Shore D |
| Wasseraufnahme (in %) | + 1.95 |

### Beispiel 6

Zur Überprüfung der Haftwerte von erfindungsgemäßen Polymerfolien an Zahnoberflächen wurde folgender Versuchsaufbau gewählt:

Die polierte Dentinoberfläche von Rinderzähnen wurde mit Ätzgel (37%iges Phosphorsäure-Gel) 15 Sekunden lang geätzt. Das Ätzgel wurd anschliessend abgespült und die Dentinoberflächen wurden getrocknet. Auf die so behandelte Oberfläche wurde 10 Sekunden lang das Dentinadhäsiv Excite® einmassiert und leicht verblasen. Auf die Oberfläche wurde ein Folienabschnitt von 4 mm Durchmesser gelegt. Ein zylindrischer Prüfstempel aus polymerisiertem Tetric® Ceram (Füllungskomposit von Ivoclar Vivadent AG) wurde auf einer Stirnfläche ebenfalls dünn mit Excite® bestrichen und auf die Folie aufgedrückt. Im letzten Präparationsschritt wurde die Versuchsanordnung 60 Sekunden lang durch den Prüfstempel hindurch mit einer Polymerisationslampe Astralis® der Fa. Ivoclar Vivadent AG bestrahlt. Die auf diese Weise hergestellten Prüfkörper wurden anschließend über 24 Stunden in Wasser bei 37 °C gelagert. Im Anschluss an die Wasserlagerung erfolgte die Messung der Haftung der Folie an dem Dentin in Anlehnung an die ISO/TS 11405 "Dental Materials - Testing of adhesion to tooth structures".

Dabei wurden zwei Reihen von je 5 Prüfkörpern gemessen, die unter Verwendung von zwei Chargen der Polyurethanfolie nach Beispiel 5 hergestellt worden waren.

| | | |
|---|---|---|
| Messreihe 1: | Durchschnittswert: | 10,6 MPa |
| | Standardabweichung: | 3,1 MPa |
| | | |
| Messreihe 2: | Durchschnittswert: | 11,0 MPa |
| | Standardabweichung: | 1,3 MPa |

Die Bruchstellen wiesen alle einen adhäsiven Bruch auf, d.h. die Folienabschnitte blieben unbeschädigt. Der erreichte Wert der Adhäsion entspricht den Anforderungen an einen Bracket-Kleber, d.h. einem Kleber zur Befestigung von Zahnklammern, der eine Haftung von 10 MPa aufweisen soll.

## Patentansprüche

1. Dentale Polymerfolie zum Beschichten von Zahnoberflächen **dadurch gekennzeichnet, dass** die Polymerfolie polymerisierbare Gruppen aufweist.

2. Dentale Polymerfolie nach Anspruch 1, bei der die polymerisierbaren Gruppen zumindest teilweise Acrylat- oder Methacrylat-Gruppen sind.

3. Dentale Polymerfolie nach Anspruch 1 oder 2, die auf der Basis von Polyurethanen, Michael-Additionsharzen oder anderen Polyaddukten aufgebaut ist.

4. Dentale Polymerfolie nach einem der Ansprüche 1 bis 3, bei der zumindest ein Teil der polymerisierbaren Gruppen radikalisch polymerisierbar ist.

5. Dentale Polymerfolie nach einem der Ansprüche 1 bis 4, bei der zumindest ein Teil der polymerisierbaren Gruppen kationisch polymerisierbar ist.

6. Dentale Polymerfolie nach einem der Ansprüche 1 bis 5, die Initiatoren für die thermische, photochemische oder redoxinduzierte Polymerisation der polymerisierbaren Gruppen enthält.

7. Dentale Polymerfolie nach Anspruch 6, die die Initiatoren in mikroverkapselter Form enthält.

8. Dentale Polymerfolie nach der Ansprüche 1 bis 7, die organische oder anorganische Füllstoffe enthält.

9. Dentale Polymerfolie nach einem der Ansprüche 1 bis 8, die einen oder mehrere Polymerisationsinhibitoren enthält.

10. Dentale Polymerfolie nach einem der Ansprüche 1 bis 9, die ein oder mehrere Antioxidantien enthält.

11. Dentale Polymerfolie nach einem der Ansprüche 1 bis 10, die ein oder mehrere Farbstoffe und/oder Pigmente enthält.

12. Dentale Polymerfolie nach einem der Ansprüche 1 bis 11, die einen Haftvermittler aufweist.

13. Dentale Polymerfolie nach einem der Ansprüche 1 bis 12, bei der die der Zahnoberfläche zugewandte Seite der Polymerfolie mit einem Haftvermittler für die Zahnhartsubstanz beschichtet ist.

14. Dentale Polymerfolie nach einem der Ansprüche 1 bis 13, die ein Antihaftadditiv aufweist.

15. Dentale Polymerfolie nach einem der Ansprüche 1 bis 14, bei der die der Zahnoberfläche abgewandte Seite der Polymerfolie mit einem Antihaftadditiv für Plaque beschichtet ist.

16. Dentale Polymerfolie nach einem der Ansprüche 1 bis 15, die ein oder mehrere Wirkstoffe und insbesondere antimikrobielle Verbindungen und/oder ein oder mehrere Antibiotika enthält.

17. Dentale Polymerfolie nach einem der Ansprüche 1 bis 16, die ablösbar auf einer Trägerfolie gehalten ist.

18. Dentale Polymerfolie nach Anspruch 17, wobei die Trägerfolie als aufblasbarer Folienbeutel ausgestaltet ist.

19. Dentale Polymerfolie nach Anspruch 17 oder 18, wobei die Trägerfolie lichtdurchlässig ist.

20. Dentale Polymerfolie gemäß einem der Ansprüche 1 bis 19, die einen optischen Aufheller enthält.

21. Verwendung einer dentalen Polymerfolie gemäß Anspruch 20 zur Beschichtung von Zahnoberflächen.

22. Verwendung der dentalen Polymerfolie gemäß einem der Ansprüche 1 bis 20 zur Herstellung einer Beschichtung von Zahnoberflächen.

23. Verwendung nach Anspruch 22, bei der
a) die Polymerfolie gemäß einem der Ansprüche 1 bis 20 auf die Zahnoberfläche aufgebracht und durch Verformen an diese angepasst wird, und
b) die polymerisierbaren Gruppen der aufgebrachten und angepassten Polymerfolie polymerisiert werden.

24. Verfahren zur Herstellung einer Beschichtung einer Zahnoberfläche, bei dem
a) die Polymerfolie gemäß einem der Ansprüche 1 bis 20 auf die Zahnoberfläche aufgebracht und durch Verformen an diese angepasst wird, und
b) die polymerisierbaren Gruppen der aufgebrachten und angepassten Polymerfolie polymerisiert werden.

25. Verfahren nach Anspruch 24, bei dem zur Beschichtung einer Zahnoberfläche im Approximalraum der Approximalraum mittels eines Spreizers gespreizt wird, die zu beschichtende Oberfläche gereinigt und mittels Ätztechnik oberfläclienkonditioniert wird, wonach ein Adhäsiv auf die so vorbereitete Zahnoberfläche aufgebracht und die polymerisierbare Polymerfolie gemäß einem der Ansprüche 1 bis 20 in den Approximalraum eingebracht und an die Zahnfläche angelegt und deren polymerisierbare Gruppen nach Anformung polymerisiert werden, um eine Beschichtung zu ergeben.

26. Kit, der eine dentale Polymerfolie gemäß einem der Ansprüche 1 bis 20 zur Versiegelung und zum mechanischen Schutz der Zahnoberfläche und ein Adhäsiv zur Fixierung der Polymerfolie auf der Zahnoberfläche aufweist.

## Claims

1. Dental polymer film for coating tooth surfaces, **characterised in that** the polymer film comprises polymerisable groups.

2. Dental polymer film according to Claim 1, in which at least some of the polymerisable groups are acrylate or methacrylate groups.

3. Dental polymer film according to Claim 1 or 2, which is based on polyurethanes, Michael addition resins or other polyadducts.

4. Dental polymer film according to one of Claims 1 to 3, in which at least a part of the polymerisable groups is radically polymerisable.

5. Dental polymer film according to one of Claims 1 to 4, in which at least a part of the polymerisable groups is cationically polymerisable.

6. Dental polymer film according to one of Claims 1 to 5, which comprises initiators for the thermal, photochemical or redox-induced polymerisation of the polymerisable groups.

7. Dental polymer film according to Claim 6, which comprises the initiators in microencapsulated form.

8. Dental polymer film according to one of Claims 1 to 7, which comprises organic or inorganic fillers.

9. Dental polymer film according to one of Claims 1 to 8, which comprises one or a plurality of polymerisation inhibitors.

10. Dental polymer film according to one of Claims 1 to 9, which comprises one or a plurality of antioxidants.

11. Dental polymer film according to one of Claims 1 to 10, which comprises one or a plurality of dyes and/or pigments.

12. Dental polymer film according to one of Claims 1 to 11, which comprises a primer.

13. Dental polymer film according to one of Claims 1 to 12, wherein the side of the polymer film facing the tooth surface is coated with a primer for the hard tooth substance.

14. Dental polymer film according to one of Claims 1 to 13, which comprises an anti-adhesive additive.

15. Dental polymer film according to one of Claims 1 to 14, wherein the side of the polymer film facing away from the tooth surface is coated with an anti-adhesive additive for plaque.

16. Dental polymer film according to one of Claims 1 to 15, which comprises one or a plurality of active substances and in particular antimicrobial compounds and/or one or a plurality of antibiotics.

17. Dental polymer film according to one of Claims 1 to 16, which is detachably held on a backing film.

18. Dental polymer film according to Claim 17, wherein the backing film is in the form of an inflatable film bag.

19. Dental polymer film according to Claim 17 or 18, wherein the backing film is translucent.

20. Dental polymer according to one of Claims 1 to 19, which comprises an optical brightener.

21. Use of a dental polymer film according to Claim 20 for coating tooth surfaces.

22. Use of the dental polymer film according to one of Claims 1 to 20 for the preparation of a coating for tooth surfaces.

23. Use according to Claim 22, in which
a) the polymer film according to one of Claims 1 to 20 is applied to the tooth surface and fitted thereto by forming, and
b) the polymerisable groups of the applied and fitted polymer film are polymerised.

24. Method for the preparation of a coating of a tooth surface, in which
a) the polymer film according to one of Claims 1 to 20 is applied to the tooth surface and fitted thereto by forming, and
b) the polymerisable groups of the applied and fitted polymer film are polymerised.

25. Method according to Claim 24, in which, for the coating of a tooth surface in the approximal space, the approximal space is expanded by means of a tooth separator, the surface to be coated is cleaned and the surface is conditioned by means of an etching technique, after which an adhesive is applied to the tooth surface thus prepared and the polymerisable polymer film according to any one of Claims 1 to 20 is introduced into the approximal space and positioned against the tooth surface and its polymerisable groups are polymerised after forming, to produce a coating.

26. Kit having a dental polymer film according to one of Claims 1 to 20 for sealing and mechanical protection of the tooth surface and an adhesive for fixing the polymer film to the tooth surface.

## Revendications

1. Film polymère dentaire destiné à recouvrir la surface des dents, **caractérisé en ce que** le film polymère présente des groupements polymérisables.

2. Film polymère dentaire selon la revendication 1, dans lequel les groupements polymérisables sont au moins en partie des groupements acrylates ou méthacrylates.

3. Film polymère dentaire selon la revendication 1 ou 2, qui est produit à partir de polyuréthanes, de résines d'addition de Michael ou d'autres polymères d'addition.

4. Film polymère dentaire selon l'une des revendications 1 à 3, dans lequel au moins une partie des groupements polymérisables sont polymérisables par voie radicalaire.

5. Film polymère dentaire selon l'une des revendications 1 à 4, dans lequel au moins une partie des groupements polymérisables sont polymérisables par voie cationique.

6. Film polymère dentaire selon l'une des revendications 1 à 5, qui contient des initiateurs de polymérisation thermique, photochimique ou induite par oxydoréduction des groupements polymérisables.

7. Film polymère dentaire selon la revendication 6, qui contient lesdits initiateurs sous une forme micro-encapsulée.

8. Film polymère dentaire selon l'une des revendications 1 à 7, qui contient des charges, organiques ou minérales.

9. Film polymère dentaire selon l'une des revendications 1 à 8, qui contient un ou plusieurs inhibiteurs de polymérisation.

10. Film polymère dentaire selon l'une des revendications 1 à 9, qui contient un ou plusieurs anti-oxydants.

11. Film polymère dentaire selon l'une des revendications 1 à 10, qui contient un ou plusieurs agents colorants et/ou pigments.

12. Film polymère dentaire selon l'une des revendications 1 à 11, qui présente une couche d'accrochage.

13. Film polymère dentaire selon l'une des revendications 1 à 12, dans lequel la face du film polymère accolée à la surface de la dent est recouverte par une couche d'accrochage pour une substance qui garantie la dureté de la dent.

14. Film polymère dentaire selon l'une des revendications 1 à 13, qui présente un additif anti-adhésif.

15. Film polymère dentaire selon l'une des revendications 1 à 14, pour lequel la face du film polymère accolée à la surface de la dent est recouverte d'un additif anti-adhésion de la plaque dentaire.

16. Film polymère dentaire selon l'une des revendications 1 à 15, qui contient une ou plusieurs substances actives, et en particulier des composés antimicrobiens et/ou un ou plusieurs antibiotiques.

17. Film polymère dentaire selon l'une des revendications 1 à 16, qui est détachable à partir d'un film support.

18. Film polymère dentaire selon la revendication 17, dans lequel le film support est façonné sous la forme d'un sac en film gonflable.

19. Film polymère dentaire selon l'une des revendications 17 ou 18, dans lequel le film support est translucide.

20. Film polymère dentaire selon l'une des revendications 1 à 19, qui contient un azurant optique.

21. Utilisation d'un film polymère dentaire selon la revendication 20, pour recouvrir la surface des dents.

22. Utilisation d'un film polymère dentaire selon l'une des revendications 1 à 20, pour la fabrication d'un revêtement pour la surface des dents.

23. Utilisation selon la revendication 22, dans laquelle :
a) le film polymère selon l'une des revendications 1 à 20 est appliqué sur la surface des dents et lui est adapté par déformation, et
b) les groupements polymérisables du film polymère appliqué, puis adapté, sont polymérisés.

24. Procédé de fabrication d'un revêtement pour la surface des dents dans lequel :
a) le film polymère selon l'une des revendications 1 à 20 est appliqué sur la surface des dents et lui est adapté par déformation, et
b) les groupements polymérisables du film polymère appliqué, puis adapté, sont polymérisés.

25. Procédé selon la revendication 24, dans lequel, pour le revêtement d'une surface de dent dans l'espace interdentaire, l'espace interdentaire est écarté au moyen d'un écarteur, la surface à recouvrir est nettoyée et conditionnée en surface au moyen des techniques d'attaque de surface, après quoi un adhésif est appliqué sur la surface dentaire ainsi préparée, le film polymère polymérisable selon une des revendications 1 à 20 est introduit dans l'espace interdentaire et appliqué sur la surface dentaire et ses groupements polymérisables sont polymérisés après déformation pour donner un revêtement.

26. Kit, qui comprend un film polymère dentaire selon l'une des revendications 1 à 20, pour l'obturation et la protection mécanique de la surface des dents, et un adhésif pour la fixation du film polymère sur la surface des dents.
